**Europäisches Patentamt**

**European Patent Office**

**Office européen des brevets**

(19)

(11) Veröffentlichungsnummer: **0 206 193**
**B1**

(12)

# EUROPÄISCHE PATENTSCHRIFT

(45) Veröffentlichungstag der Patentschrift:
23.08.89

(51) Int. Cl.⁴: **B01J 27/18, B01J 23/84,**
**B01J 23/85, B01J 23/06,**
**B01J 23/88**

(21) Anmeldenummer: 86108178.4

(22) Anmeldetag: 14.06.86

(54) Wabenförmiger Katalysator, seine Herstellung und seine Verwendung.

(30) Priorität: 19.06.85 DE 3521766

(43) Veröffentlichungstag der Anmeldung:
30.12.86 Patentblatt 86/52

(45) Bekanntmachung des Hinweises auf die Patenterteilung:
23.08.89 Patentblatt 89/34

(84) Benannte Vertragsstaaten:
AT BE CH DE FR GB IT LI NL

(56) Entgegenhaltungen:
EP-A- 0 055 533
EP-A- 0 072 439
FR-A- 2 147 051
FR-A- 2 231 669
US-A- 4 021 370

Die Akte enthält technische Angaben, die nach dem
Eingang der Anmeldung eingereicht wurden und die
nicht in dieser Patentschrift enthalten sind.

(73) Patentinhaber: BASF Aktiengesellschaft,
Carl-Bosch-Strasse 38, D-6700 Ludwigshafen(DE)

(72) Erfinder: Hoelderich, Wolfgang, Dr., Mannheimer
Strasse 18 c, D-6710 Frankenthal(DE)
Erfinder: Biffar, Werner, Dr., Fontanesistrasse 11,
D-6710 Frankenthal(DE)
Erfinder: Irgang, Matthias, Dr., Andreas-Hofer-Weg 41,
D-6900 Heidelberg(DE)
Erfinder: Mross, Wolf Dieter, Dr.,
Anselm-Feuerbach-Strasse 21, D-6710 Frankenthal(DE)
Erfinder: Kroener, Michael, Dr., Eislebener Weg 8,
D-6800 Mannheim 31(DE)
Erfinder: Ambach, Eberhard, Dr., Carl-Bosch-Strasse 7,
D-6703 Limburgerhof(DE)

## Beschreibung

Die Verwendung der Wabenkörper ist vielfältig, z.B. als Filtermaterial, als Füllmaterial in Waschtürmen und Destillationskolonnen, als Hitzeschilder u.a.m. Es ist auch bekannt, Wabenkörper als Katalysatoren oder Katalysatorträger einzusetzen. So hat man Wabenkörper z.B. bei der heterogenen Katalyse, insbesondere zur Entgiftung und katalytischen Verbrennung von Motor- und Kraftwerksabgasen eingesetzt (DE-OS 24 43 262 und DE-AS 20 45 488).

Zur Herstellung von Wabenkörpern hat man in der Regel keramische Materialien verwendet, z.B. Cordierit und Mullit (US-PS 3 112 184, DE-OS 22 54 563, DE-OS 21 52 490). Die Wabenkörper sind entweder direkt mit einer katalytisch aktiven Masse beschichtet, oder einer besonderen Auflage, auf die die eigentlich katalytische aktive Masse aufgetragen wird.

Die Erfindung betrifft einen wabenförmigen Katalysator bestehend aus 30 bis 95 Gew.% einer Eisenverbindung, berechnet als $Fe_2O_3$ und 0,1 bis 60 Gew.%, bevorzugt 0,1 bis 20 Gew.% einer Aluminium-, Cer- und/oder Chromverbindung berechnet als $Al_2O_3$, $CeO_2$, $Cr_2O_3$ und/oder 1 bis 50 Gew.% einer Alkaliverbindung, insbesondere Kalium berechnet als $K_2O$ und/oder 0,2 bis 20 Gew.% eines Elements der 6. Nebengruppe berechnet als $MeO_3$, bevorzugt $MoO_3$ und $WO_3$ und/oder 0,1 bis 20 Gew.% einer Verbindung des Vanadins berechnet als $V_2O_5$ und/oder 0,1 bis 20 Gew.% einer Verbindung des Phosphors berechnet als $P_2O_5$ sowie ein Verfahren zu seiner Herstellung und die Verwendung derselben.

Bei den erfindungsgemäßen Wabenkörpern handelt es sich zumeist um sogenannte Vollkatalysatoren, d.h. der Körper besteht durch und durch aus katalytisch aktiver Masse im Gegensatz zu den bekannten Wabenkatalysatoren, bei denen die Aktivkomponente auf ein Trägermaterial aufgebracht wird. Die Herstellung der Wabenkörper ist nicht ohne Schwierigkeiten zu erreichen und erfordert ein hohes Maß an technischem Können. Um so überraschender war es, daß die erfindungsgemäßen Wabenkörper aus dem Gemenge der Ausgangsstoffe z.B. von Metalloxiden also aus einem nicht keramischen Material, das sich normalerweise aufgrund seines rheologischen Verhaltens besonders gut für die Extrusion eignet, herstellen lassen.

In der EP-Anm. 0 072 439 ist ein Dehydrierungskatalysator ähnlicher Zusammensetzung beschrieben, der kein Ce aber 0,1 bis 5 Gew.% einer Lithiumverbindung enthält und der sich von dem erfindungsgemäßen Katalysator vor allem durch die Verformung der Katalysatormasse zu zylindrischen Ringen oder sternförmigen Strangpreßlingen unterscheidet.

Die Ausgangsgemische für die Herstellung der erfindungsgemäßen Katalysatoren setzen sich aus mehreren Komponenten zusammen:

- Die Komponente a) repräsentiert mindestens eine Eisenverbindung, die bevorzugt in einem Anteil von 30 bis 95 Gew.%, berechnet als $Fe_2O_3$, im Katalysator zugegen ist. Als Eisenverbindung zur Herstellung des Katalysators kann man von den verschiedenen Eisenoxiden bzw. Eisenoxidhydraten ausgehen. Geeignet sind z.B. die rotbraunen oder gelben bzw. schwarzen Eisenoxidpigmente.

- Als Komponente b) wird entweder eine Chrom-, Cer- und/oder eine Aluminiumverbindung in einem Anteil von 0,1 bis 60 Gew.%, berechnet als $Cr_2O_3$, $CeO_2$, $Al_2O_3$, bevorzugt 0,1 bis 20 Gew.% angewendet. Diese Komponenten wirken im Katalysator als Strukturstabilisatoren und verlängern die Standzeit der Katalysatormischung.

- Als Komponente c) erhält das Ausgangsgemisch 1 bis 50 Gew.%, vorzugsweise 3 bis 40 Gew.% einer Alkaliverbindung, insbesondere einer Kaliumverbindung, berechnet als $K_2O$. Die Art der angewendeten Kaliumverbindung richtet sich nach der Art der Herstellung. Es werden vorzugsweise solche Verbindungen gewählt, die beim Calcinieren des Katalysators sich zum Teil zu $K_2O$ zersetzen; genannt seien das Hydroxid, das Carbonat, das Bicarbonat, das Carboxilat, das Borat oder die Phosphate des Kaliums. Bevorzugt wird Kalium als Kalilauge zur Unterstützung des Anmaischens bei der Herstellung des Wabenkörpers in die Mischung der übrigen Katalysatorbestandteile eingemischt. Aber auch Verbindungen verschiedener Alkalimetalle können gemeinsam angewendet werden.

- Als Komponente d) kann eine Verbindung eines Elements der 6. Nebengruppe des Periodensystem berechnet als $MeO_3$, angewendet werden. Besonders bevorzugt wird diese Verbindung in einer Menge von 0,2 bis 20 Gew.%. Insbesondere werden Verbindungen der Elemente Molybdän ($MoO_3$) und Wolfram ($WO_3$) angewendet. Die Elemente werden zweckmäßig in Form der Sauerstoffsäuren bzw. deren Ammoniumsalze oder Kaliumsalze in der Oxidationsstufe +6 für die Herstellung des Katalysators verwendet, da sich diese Verbindungen beim Calcinieren leicht zersetzen. Besonders geeignet sind die Heteropolysäuren von Molybdän und Wolfram mit Phosphorsäure, z.B. die Wolframatophosphorsäure bzw. die Molybdatophosphorsäure.

Anstelle der Komponente d) oder zusammen mit dieser kann der erfindungsgemäße Katalysator 0,1 bis 20 Gew.%, mindestens eine Verbindung des Vanadins oder des Phosphors, berechnet als $V_2O_5$ bzw. $P_2O_5$ aufweisen (Komponente e). Bevorzugt werden von diesen Verbindungen Mengen von 0,1 bis 20 Gew.%. Als Verbindungen des Vanadins kommen insbesondere die Oxide, die Ammoniumsalze oder die Kaliumsalze der höchsten Oxidationsstufe in Betracht, z.B. $V_2O_5$, $NaVO_3$ bzw. $KVO_3$. Falls sowohl eine Verbindung des Vanadins als auch des Phosphors verwendet wird, werden bevorzugt die Heteropolysäuren des Vanadins mit Phosphorsäure, z.B. die Vanadatophosphorsäure oder ihr Ammonium- oder Kaliumsalz ausgewählt. Bei der Wahl der Kalisalze dieser Heteropolysäuren sind die entsprechenden Abschläge für die Komponente b) zu berücksichtigen. Falls Phosphor alleine verwendet wird, wird dieser in Form des Oxids, der Säure, bzw. der Ammoniumsalze oder der Kaliumsalze angewendet oder gemeinsam mit der Komponente d) als Heteropolysäure eingebracht.

Der erfindungsgemäße Katalysator kann gegebenenfalls auch noch weitere Zusätze enthalten. Beispielsweise seien genannt: Elemente der 8. Nebengruppe wie Co, Ni, Elemente der 1. Nebengruppe wie Cu, Ag, Elemente der 2. Nebengruppe wie Zn, Cd, Elemente der 4. Nebengruppe wie Ti, Zr, Elemente der 7. Nebengruppe wie Mn, Seltenen Erdmetalle wie La, Pr, Nd, Actinide wie Th, U, Erdalkalimetalle wie Mg, Ca, Sr, Ba und Elemente der 4. und 5. Hauptgruppe wie Pb und Bi.

Bei der Herstellung der erfindungsgemäßen Katalysatoren geht man in der Regel aus von einer Mischung der oben genannten Komponenten die in der Regel mit Wasser, ggf. unter Zusatz von Kalilauge sowie unter Zusatz von Extrudierhilfsmitteln wie Stärke, Methylcellulose, Graphit, Polyethylenglykole, Polyvinylalkohole, Polyvinylpyrrolidone, Polyacrylester, Stearinsäure und deren Metallsalze, Naphthalin, Ammoniak, Ameisensäure, Oxalsäure und Salpetersäure entweder mit einem porositätsverbessernden Mittel oder Gleitmittel oder Peptisierungsmittel oder zunächst geknetet und anschließend verformt wird. Bevorzugt wird dabei die Knetung mit anschließender Verformung, insbesondere durch Extrudieren. Die bei der Extrusion erhaltenen Formlinge werden zunächst bei Temperaturen im Bereich von Raumtemperatur bis 160°C getrocknet und danach bei Temperaturen im Bereich von 300 bis 1000°C calciniert, wobei auch eine stufenweise Vorcalcination von Vorteil sein kann.

Eine Ausführungsform besteht darin, daß man das Gemenge der Ausgangsstoffe mit Wasser anteigt und die Maische auf ein organisches Material aufbringt und nach Verformung zu Wabenkörpern die organischen Bestandteile herausbrennt.

Die erfindungsgemäßen Katalysatoren können verschiedene Wabenformen aufweisen. Die Grundfläche kann kreisförmig, oval oder polygonal sein. Die Länge kann ein Vielfaches des Querschnitts erreichen. Die Körper sind von einer Vielzahl parallel verlaufender Kanäle durchzogen, deren Grundfläche ebenfalls kreisförmig, oval, geschwungen oder eckig sein kann. Entscheidend für einen niedrigen Druckverlust ist der freie Querschnitt der Anströmfläche, der über 40 % erreichen soll. Die Festigkeit des Wabenkörpers limitiert den freien Querschnitt.

Die äußeren Dimensionen der Formkörper erreichen üblicherweise bis zu 150 mm Kantenlänge der Grundfläche bei Quadern und etwa gleichen Durchmesser der Grundfläche bei Zylindern. Die Länge der Körper über den Grundflächen kann über 1000 mm liegen. Die lichte Weite der inneren Kanäle variiert zwischen 0,5 und 20 mm. Die Stärke der inneren Trennwände liegt üblicherweise zwischen 0,1 und 5 mm.

Auch den Wabenkörpern ähnliche Formkörper können eingesetzt werden. Die Kanäle dieser Formkörper verlaufen nicht parallel zur zentralen Achse sondern schräg dazu durch den Körper, z.B. nach dem Prinzip der statischen Mischer, z.B. Kerapak®-Formen. Mit diesen Formkörpern wird die Vermischung der Reaktionsgase quer zur zentralen Achse erhöht, wodurch ein erhöhter Wärmeaustausch zwischen Reaktorwand und Innenraum eintritt. Den Wabenkörpern ähnlich bestehen auch solche Formkörper aus Katalysator-Vollmaterial.

Für katalytische Reaktionen mit starker Wärmetönung empfiehlt es sich zur Förderung des Wärmeaustausches mehrere Wabenkörper mit parallel verlaufenden Kanälen im Reaktor so anzuordnen, daß sich zwischen den einzelnen Wabenkörpern Mischstrecken oder statische Mischer aus keramischen oder metallischen Materialien (inert oder katalytisch aktiv) befinden, um eine Durchmischung der Reaktionsgase zu gewährleisten, bzw. um gezielt die an der Reaktorwand strömenden Gase in das Reaktorinnere und die innenströmenden Gase nach außen abzulenken. Die erfindungsgemäßen Katalysatoren können allgemein bei organisch chemischen Reaktionen Verwendung finden, z.B. bei Dehydrierungen und Dehydrocyanisierungen und Alkoholabspaltung. Man kann die wabenförmigen Katalysatoren aber auch bei der Hochtemperaturkonvertierung anwenden.

Vorteile der erfindungsgemäßen Wabenkatalysatoren sind z.B. bei hochselektiven Dehydrierreaktionen.

a) Die günstigen kinetischen Voraussetzungen - große Oberfläche bei kurzen Diffusionswegen im Porensystem -, die zu einer deutlichen Aktivitäts- und Selektivitätssteigerung führen.

b) Der geringe Druckverlust über das Katalysatorbett, der höhere Mengenströme pro Katalysatorvolumen und auch Reaktionen unterhalb Atmosphärendruck zuläßt.

c) Ein modularer Einsatz von Wabenkörpern erlaubt den raschen Ein- und Ausbau des Katalysators, auch schneller Katalysatorteilwechsel ist möglich.

d) Die in einer Festbettschüttung auftretenden Pelletbewegungen unterbleiben, wodurch ein mechanischer Abrieb weitgehend unterbunden wird.

e) Durch die handliche Form das Katalsators wird Transport, Aufarbeitung oder Regenerierung außerhalb des Reaktors und die endgültige Entsorgung und Endlagerung des Katalysators erleichtert, insbesondere, wenn bei einer anderen Aufarbeitung gesundheitsgefährende Stäube auftreten können.

Die erfindungsgemäßen Katalysatoren und deren Anwendung werden in den folgenden Beispielen belegt.

Beispiel 1

Katalysator zur thermischen Dehydrierung von Ethylbenzol zu Styrol.

10.000 g $\alpha$-FeOOH, 1.596 g $K_2CO_3$, 190 g $Cr_2O_3$, 146 g $V_2O_5$, 115 g $WO_3$ und 300 g Stärke werden gemischt und mit Wasser angemaischt. Nach 3 h Knetzeit wird die plastische Masse zu Wabenkörpern extrudiert. Die quadratische Grundfläche der erhaltenen quaderförmigen Körper hat Kanten von 5 cm Länge. Die Länge der Quader beträgt 40 cm. Die 121 inneren Kanäle haben eine quadratische Stirnfläche mit 0,36 mm Kantenlänge. Die Dicke der inneren Trennwände beträgt 0,55 mm. Die Wabenkörper werden 2 Tage bei ansteigender Temperatur bis 150°C getrocknet. Anschließend werden die Formkörper 5 h bei 500°C und 1 h bei 850°C calciniert.

Der Test des Katalysators erfolgt in einem elektrisch beheizten Modellreaktor. Die Rohrgeometrie des Reaktors wird dem Formkörper passend gewählt.

Über den Katalysator werden je Stunde 1 kg Ethylbenzol und 1,5 kg Wasser nach Verdampfung bei 610°C geleitet. Dies entspricht einer Belastung von 1 kg Ethylbenzol pro kg Katalysator und Stunde.

Unter den genannten Bedingungen wird nach 100 h Laufzeit ein Umsatz von 52,9 % des eingeleiteten Ethylbenzols erreicht. Vom umgesetzten Ethylbenzol werden 94,8 % in Styrol umgewandelt.

Beispiel 2

Katalysator zur thermischen Dehydrocyanisierung von Formylalaninnitril zu Vinylformamid

3000 g $\alpha$-FeOOH (Eisengelb), 27,6 g $V_2O_5$, 78 g $CrO_3$, 81 g $WO_3$, 34,5 g $Li_2CO_3$ werden gut miteinander gemischt. Danach wird dieses Gemenge mit 329 g KOH, 168 g Kartoffelstärke und 28 g Methylcellulose gelöst in 1 l $H_2O$ angemaischt und im Kneter 1 h verdichtet. Die plastische Masse wird zu Wabenkörpern extrudiert. Die quadratische Grundfläche des erhaltenen quaderförmigen Körpers hat 5 cm Kantenlänge.

Die Länge des Quaders selbst beträgt 12 cm. Die 36 inneren Kanäle haben eine quadratische Stirnfläche mit 6 mm lichter Kantenlänge. Die Stärke der inneren Trennwände weist 1,5 mm auf. Dieser Wabenkörper wird 3 Tage bei Raumtemperatur getrocknet und während 2 h bei ansteigender Temperatur bis 150°C/4 h getrocknet. Anschließend wird der Formkörper 1 h bei 500°C und mit ansteigender Temperatur bis 850°C/1 h calciniert.

10 solcher Wabenkörper werden in einen elektrisch beheizten Rohrreaktor aus Edelstahl, dessen Geometrie dem Formkörper angepaßt ist, eingebaut. Jeder der Wabenkörper ist durch je einen 50 mm langen statischen Mischer aus keramischen Material z.B. Kerapak® vom nächsten Wabenkörper getrennt. Dem Pyrolyserohr ist ein Dünnschichtverdampfer vorgeschaltet. Während der Reaktion werden eine Reaktionstemperatur von 460°C und ein Druck von 30 mbar aufrechterhalten. Die Belastung beträgt 0,8 kg Formylalaninnitril/kg Katalysator × h. Gleichzeitig werden 30 l Luft/h mitgeführt. Es wird ein Umsatz von 97 % und eine Selektivität von 93 % erzielt.

Beispiel 3

Katalysator zur Hochtemperaturkonvertierung

25.200 g $FeSO_4 \times 7 H_2O$ und 473 g $CrO_3$ werden in Wasser gelöst und mit konz. $NH_3$-Lösung unter Einleiten von Luft umgesetzt. Der erhaltene Niederschlag wird filtriert, gewaschen und mit 44,9 g $NH_4VO_3$, 250 g Stärke und wenig Wasser angemaischt. Nach 3 h Knetzeit wird die plastische Masse zu Wabenkörpern analog Beispiel 1 extrudiert.

Der Test des Katalysators erfolgt in einem elektrisch beheizten Modellreaktor. Die Rohrgeometrie des Reaktors wird dem Formkörper angepaßt. Die Testbedingungen sind:

| Katalysatormenge: | 1 L # 1 Wabenkörper |
|---|---|
| Trockengaszusammensetzung: | 60 Vol.-% $H_2$ |
| | 20 Vol.-% CO |
| | 20 Vol.-% $N_2$ |
| Durchsätze: | 3000 Nl × h⁻¹ Wasserstoff |
| | 1000 Nl × h⁻¹ Kohlenmonoxid |
| | 1000 Nl × h⁻¹ Stickstoff |
| | 3480 Nl × h⁻¹ Wasserdampf |
| Belastung (bez. auf Trockengas): | 5000 h⁻¹ |
| Druck: | 30 bar |

Die gaschromatographische Auswertung ergibt bei einer Reaktionstemperatur von 330°C nach 1 Tag Laufzeit 91 % CO-Umsatz.

**Patentansprüche**

1. Wabenförmiger Katalysator, bestehend aus 30 bis 95 Gew.-% einer Eisenverbindung, berechnet als $Fe_2O_3$ und 0,1 bis 60 Gew.-%, einer Aluminium- und/oder Cer- und/oder Chromverbindung berechnet als $Al_2O_3$, $CeO_2$, $Cr_2O_3$, 1 bis 50 Gew.-% einer Alkaliverbindung, insbesondere Kalium berechnet als $K_2O$ und 0,2 bis 20 Gew.-% eines Elements der 6. Nebengruppe berechnet als $MeO_3$, bevorzugt $MoO_3$ und $WO_3$ und/oder 0,1 bis 20 Gew.-% einer Verbindung des Vanadins berechnet als $V_2O_5$ und/oder 0,1 bis 20 Gew.% einer Verbindung des Phosphors berechnet als $P_2O_5$.

2. Verfahren zur Herstellung eines wabenförmigen Katalysators nach Anspruch 1, dadurch gekennzeichnet, daß man das Gemenge der Ausgangsstoffe unter Anteigen mit Wasser, gegebenenfalls unter Zusatz eines Extrudierhilfsmittels, in Wabenform extrudiert.

3. Verfahren zur Herstellung eines wabenförmigen Katalysators nach Anspruch 1, dadurch gekennzeichnet, daß man das Gemenge der Ausgangsstoffe mit Wasser anteigt und die Maische auf ein organisches Material aufbringt und nach Verformung zu Wabenkörpern die organischen Bestandteile herausbrennt.

4. Verwendung des wabenförmigen Katalysators nach Anspruch 1 bis 3 für organisch-chemische Reaktionen.

5. Verwendung des wabenförmigen Katalysators nach Anspruch 1 bis 3 als Dehydrocyanisierungskatalysator.

6. Verwendung des wabenförmigen Katalysators nach Anspruch 1 bis 3 als Dehydrocyanisierungskatalysator.

7. Verwendung des wabenförmigen Katalysators nach Anspruch 1 bis 3 als Hochtemperaturkonvertierungskatalysator.

## Claims

1. A honeycomb catalyst consisting of from 30 to 95% by weight, calculated as $Fe_2O_3$, of an iron compound and from 0.1 to 60% by weight, calculated as $Al_2O_3$, $CeO_2$ or $Cr_2O_3$, of an aluminum and/or cerium and/or chromium compound, from 1 to 50% by weight of an alkali metal compound, in particular potassium, calculated as $K_2O$, and from 0.2 to 20% by weight, calculated as $MeO_3$, of an element of subgroup 6, preferably $MoO_3$ and $WO_3$, and/or from 0.1 to 20% by weight, calculated as $V_2O_5$, of a compound of vanadium and/or from 0.1 to 20% by weight, calculated as $P_2O_5$, of a compound of phosphorus.

2. A process for the preparation of a honeycomb catalyst as claimed in claim 1, wherein a mixture of the starting materials is kneaded with water, with or without addition of an extrusion assistant, and the product is extruded to give a honeycomb.

3. A process for the preparation of a honeycomb catalyst as claimed in claim 1, wherein a mixture of starting materials is kneaded with water, the slurry is applied onto an organic material, molding is carried out to give honeycombs, and the organic components are then burned out.

4. Use of a honeycomb catalyst as claimed in claim 1 or 2 or 3 for organic chemical reactions.

5. Use of a honeycomb catalyst as claimed in claim 1 or 2 or 3 as a dehydrogenation catalyst.

6. Use of a honeycomb catalyst as claimed in claim 1 or 2 or 3 as a dehydrocyanization catalyst.

7. Use of a honeycomb catalyst as claimed in claim 1 or 2 or 3 as a high temperature conversion catalyst.

## Revendications

1. Catalyseur en nid d'abeilles, contenant 30 à 95% en poids d'un composé du fer, exprimé en $Fe_2O_3$, et 0,1 à 60% en poids d'un composé de l'aluminium et/ou du cérium et/ou du chrome, exprimé en $Al_2O_3$, $CeO_2$, $Cr_2O_3$, 1 à 50% en poids d'un composé d'un métal alcalin, en particulier du potassium exprimé en $K_2O$, et 0,2 à 20% en poids d'un élément du groupe VIb exprimé en $MeO_3$, de préférence $MoO_3$ et $WO_3$, et/ou 0,1 à 20% en poids d'un composé du vanadium exprimé en $V_2O_5$ et/ou 0,1 à 20% en poids d'un composé du phosphore exprimé en $P_2O_5$.

2. Procédé de préparation d'un catalyseur en nid d'abeilles selon la revendication 1, caractérisé en ce qu'on extrude en nid d'abeilles le mélange des substances de départ sous forme de pâte avec de l'eau, éventuellement avec addition d'un adjuvant d'extrusion.

3. Procédé de préparation d'un catalyseur en nid d'abeilles selon la revendication 1, caractérisé en ce qu'on met sous forme de pâte avec de l'eau le mélange des substances de départ, on dépose la pâte sur un matériau organique et, après formation des produits en nid d'abeilles, on élimine par combustion les parties organiques.

4. Utilisation du catalyseur en nid d'abeilles selon les revendications 1 à 3, pour des réactions organochimiques.

5. Utilisation du catalyseur en nid d'abeilles selon les revendications 1 à 3, comme catalyseur de déshydrogénation.

6. Utilisation du catalyseur en nid d'abeilles selon les revendications 1 à 3, comme catalyseur de décyanhydratation.

7. Utilisation du catalyseur en nid d'abeilles selon les revendications 1 à 3, comme catalyseur de conversion à températures élevées.